# EUROPEAN PATENT APPLICATION

(11) **EP 2 893 905 A1**
(43) Date of publication of application: **15.07.2015**
(21) Application number: 15275003.0
(22) Date of filing: 07.01.2015
(51) Int. Cl.: A61F 2/24, A61L 27/36, A61L 27/56

(54) **Heart valve suppport ring**

(30) Priority: 08.01.2014 US 201461924870 P
(71) Applicant: Cook Biotech, Inc., West Lafayette, IN 47906 (US); Cook Medical Technologies LLC, Bloomington, IN 47402 (US)
(72) Inventor: Johnson, Chad E., West Lafayette, Indiana 47906 (US); McIntosh, Charles L., Silver Spring, Maryland 20903 (US)
(74) Representative: Williams Powell

(57) **Abstract**

This disclosure relates to prosthetic heart valve support cuffs (110) that are comprised of a foamy material. Such support cuffs (110) may be configured with support frames (130) configured to maintain an improved implantation site for percutaneously delivered heart valves. The support cuff (110) may either be implanted prior to implantation of a heart valve or concurrently with the heart valve. The foamy material is configured to compress and expand upon liquid contact to substantially seal the perimeter of an opening to a heart.

## Description

### BACKGROUND

This disclosure concerns apparatuses and methods useful for preventing a perivalvular leak associated with an implanted heart valve. In particular, apparatus and methods are disclosed for sealing and to promote healing of the area between the natural valve annulus and the wall of the implanted valve.

The human heart is a section of highly muscularized vasculature defining four chambers: the right atrium, the right ventricle, the left atrium, and left the ventricle. Each chamber is associated with a one-way valve for controlling the direction of blood flow. Blood flow is driven by pressure created by regular contraction (systole) and relaxation (diastole) of cardiac muscle. Contraction of the muscles surrounding the ventricles increases the pressure within the ventricular chamber driving blood through the one-way exit valve. When the chamber relaxes negative pressure is created which causes blood to flow in from the associated atria through the one-way entrance valve.

Deoxygenated blood is pulled into the heart via the right atrium, through the tricuspid valve, and into the right ventricle. During systole, ventricular pressure increases pushing deoxygenated blood out of the right atrium through the pulmonary valve. Blood is oxygenated in the pulmonary system and collects in the left ventricle. During diastole, oxygenated blood is pulled from the left atrium, through the mitral (or bicuspid) valve, and into the left ventricle. During systole, ventricular pressure increases pushing oxygenated blood out of the left ventricle through the aortic valve.

Each of the valves resists retrograde flow, for example: from the ventricles back to their corresponding atria or from the arteries back to their corresponding ventricles. Each valve is surrounded by an annulus comprising a dense fibrous ring which supports the valve. The collagenous ring of the annulus provides an anchor for the valve leaflets which must resist backflow pressure.

About 5 million Americans are diagnosed with heart valve disease every year. There are several types of heart valve disease, for example: valvular stenosis, and valvular insufficiency. Valvular stenosis occurs when the valve leaflets become stiff narrowing the valve opening and thus reducing the volume of blood that can pass through it. Valvular insufficiency occurs when the valve leaflets do not properly close allowing blood to flow retrograde across the valve.

It is well known to treat heart valve disease by replacing a diseased or damaged valve. The procedure, whether surgical or percutaneous, involves reshaping the annulus to receive a prosthetic heart valve (either bioprosthetic or mechanical). After implantation perivalvular leaks can develop which allow backflow around the implanted valve between patient tissue and the implanted valve. These leaks can occur, for example, because the reshaped annulus is not round or elliptical conforming to the shape of the implanted valve. Another cause may be an accumulation of calcium plaques on natural valve surfaces. When the annulus is expanded, in preparation for implantation, calcium deposits can fracture creating irregularities in the implantation site. Perivalvular leaks can result in diminished valve function, loss of blood pressure, increased stress on the heart, and hemolytic anemia. A need therefore exist for improved and/or alternate devices and techniques for sealing an implanted heart valve to the annulus.

### SUMMARY

The present invention seeks to provide an improved device for reducing perivalvular flow.

According to an aspect of the present invention, there is provided a device for reducing perivalvular flow as specified in claim 1.

According to another aspect of the present invention, there is provided a device for reducing perivalvular flow as specified in claim 14.

In certain aspects the present invention provides unique medical devices that can effectively seal the perimeter of an opening to a heart and receive a percutaneously deliverable heart valve. In accordance with some forms of the invention, such medical devices are configured to utilize an expandable foam material to seal the implantation area and prevent perivalvular leaks. Accordingly, in one embodiment, the present disclosure provides a device that includes a support frame, a cuff, and a lumen. The cuff comprises a tubular wall composed of a foam material with a minimal thickness of about 1mm. The tubular wall is deformable to a compressed state and is swellable upon contact with a liquid to a swelled state. The lumen is defined by the inner surface of the cuff. The lumen is sized and configured to receive an implantable heart valve. The support frame supports the lumen and has a hoop strength sufficient to maintain a substantially circular lumen cross-section.

In accordance with certain inventive variants, the foam material is configured to expand to at least 3x (three times) by volume in de-ionized water. In one form the foam material is a synthetic polymer. In one form the foam material is an extracellular matrix foam material. In certain aspects the extracellular matrix foam material is crosslinked. In one embodiment the tubular wall is sized and configured such that the cuff pushes against patient tissue when swelled.

The device may be expandable, from a collapsed position configured for passage through a cannulated device, to an expanded position configured for placement within an opening to a heart. In one form the support frame is configured to push against the inner surface of the tubular wall. In one form the support frame is embedded within the foam material. In one form the support frame is composed of nitinol. In another form the support frame is bioresorbable.

The device may include retention elements configured to secure the device in an opening to a heart. In some forms the retention elements comprise barbs on the outer surface of the tubular wall.

In another embodiment, the disclosure provides a device that includes a cuff and a heart valve. The cuff has an outer surface, and an inner surface defining a lumen. The cuff comprises a foam material which is deformable to a compressed state and swellable upon contact with a liquid to a swelled state. The cuff is sized and configured such that in the swelled state the outer surface pushes against the walls of an opening to a heart. The heart valve is receivable within the lumen. In one form the foam material is configured to expand to at least 3x by volume in de-ionized water. In one aspect, the foam material comprises an extracellular matrix foam material.

In accordance with certain inventive variants, the device further includes a support frame configured to support the lumen prior to insertion of the heart valve. In one form the support frame is composed of nitinol. In another form the support frame is bioresorbable.

In one aspect the device may include retention elements configured to secure the device in an opening to a heart. In some forms the retention elements comprise barbs on the outer surface of the tubular wall.

In another embodiment, the disclosure provides a method for preventing blood flow around an implanted heart valve. Such method comprises implanting a cuff comprising a foam material within an opening to a heart. The cuff comprises a tubular wall defined by an inner lumen and an outer surface. The cuff is configured to expand upon contact with a liquid such that the outer surface contacts the opening. The lumen is sized and configured to receive a heart valve.

In some forms the method includes attaching a heart valve sized and configured to fit within the lumen. In certain embodiments the heart valve is attached after the cuff is implanted. In certain embodiments the heart valve is attached before the cuff is implanted.

Additional embodiments, as well as features and advantages of embodiments of the invention, will be apparent from the description herein.

### BRIEF DESCRIPTION OF THE DRAWINGS

Embodiments of the present invention are described below, by way of example only, with reference to the accompanying drawings, in which:
Figure 1 provides a perspective view of one embodiment of the disclosed device deployed within a heart valve opening;
Figure 2 provides a cut-away perspective view of one embodiment of the disclosed device in which the support frame rests against the internal surface of the cuff;
Figure 3 provides a cut-away perspective view of one embodiment of the disclosed device in which the support frame is disposed within the cuff;
Figure 4A provides a top view of an opening to a heart before implantation of the disclosed device;
Figure 4B provides a top view of an opening to a heart into which one embodiment of the disclosed device, in a compressed state, has been inserted;
Figure 4C provides a top view of an opening to a heart into which one embodiment of the disclosed device has been inserted and allowed to expand to a swelled state;
Figure 4D provides a top view of an opening to a heart into which one embodiment of the disclosed device has been inserted, the illustrated embodiment includes a heart valve;
Figure 5A provides a top view of one embodiment of the disclosed device in a collapsed frame position for passage through a cannulated device;
Figure 5B provides a top view of one embodiment of the disclosed device in an expanded frame position, in which the cuff is in a compressed state;
Figure 5C provides a top view of one embodiment of the disclosed device in an expanded frame position, in which the cuff is in a swelled state; and
Figure 6 provides a perspective view of one embodiment of the disclosed device.

### DESCRIPTION OF THE SELECTED EMBODIMENTS

For the purposes of promoting an understanding of the principles of the disclosure, reference will now be made to the embodiments illustrated in the drawings and specific language will be used to describe the same. It will nevertheless be understood that no limitation of the scope of the claims is thereby intended, and alterations and modifications in the illustrated device, and further applications of the principles of the disclosure as illustrated therein are herein contemplated as would normally occur to one skilled in the art to which the disclosure relates.

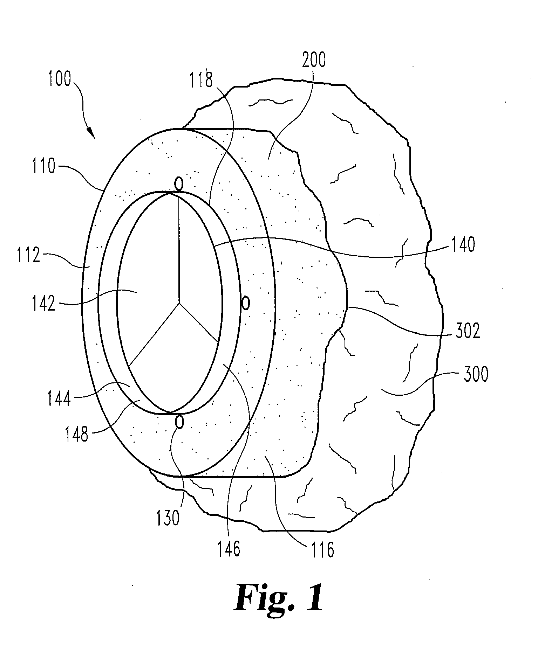

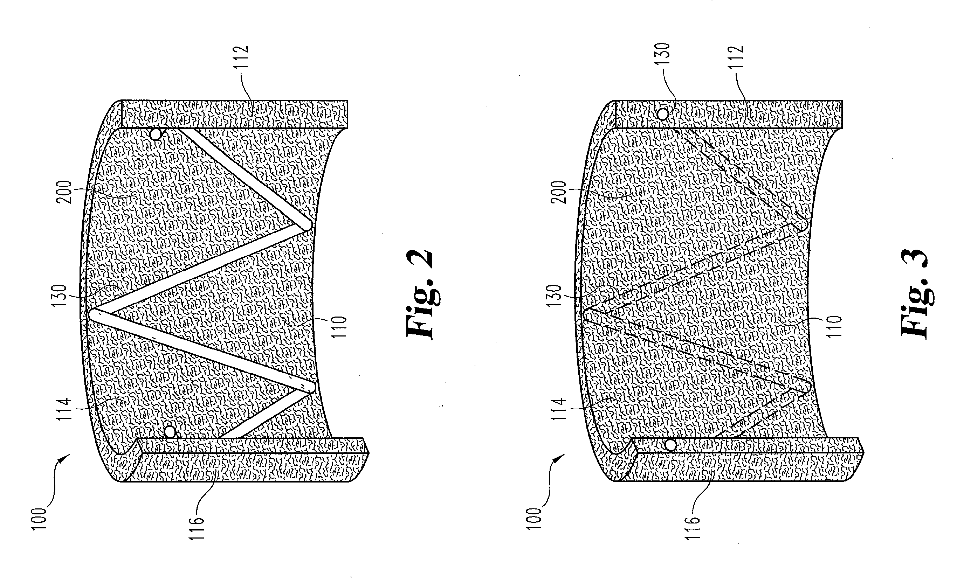

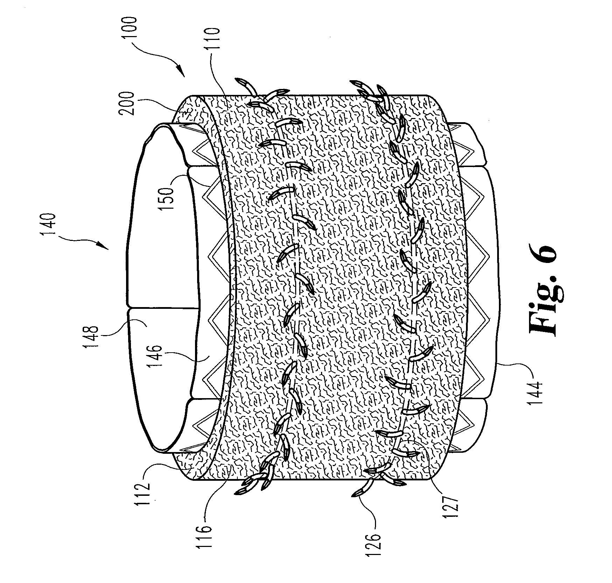

FIG. 1 is a perspective view of device **100** deployed within an opening to a heart **302** in patient tissue **300.** In the illustrated embodiment, device **100** includes cuff **110,** support frame **130** and lumen **118.** In some forms, cuff **110** comprises a tubular wall **112,** said tubular wall may be composed of a foam material **200** having an inner surface **114** (see Figures 2-3) and an outer surface **116.** Lumen **118** is supported by support frame **130** such that lumen **118** maintains a substantially circular cross section sized and configured to receive a heart valve **140.** In some forms heart valve **140** may include valve leaflets **142** and valve wall **144.** Valve wall **144** having an internal valve surface **148** and an external valve surface **146.** In some forms heart valve **140** includes a heart valve stent **150** (see Figure 6).

In some forms, support frame **130** is configured to push against inner surface **114** as shown in FIG. 2. In other forms, support frame **130** is embedded within foam material **200** as shown in FIG. 3. It is also envisioned that support frame **130** may be covered by a sheet material such as extracellular matrix sheet material or a polymer sheet material. Support frame **130** may be composed of a resiliently deformable material such as nitinol. In some forms, support frame **130** is bioresorbable and may comprise material such as poly lactic-*co*-glycolic acid.

In some forms, foam material **200** may comprise a permanent or absorbable synthetic foam material. Non-limiting examples include: polyvinyl alcohol (PVA) or polyurethane. Foam material **200** may also comprise an expandable extracellular matrix material that is formed into the appropriate shape and either cross-linked or not. Accordingly, in some forms the foam material is configured to expand at least 3 times by volume when immersed in de-ionized water. In some forms, the foam material may expand up to 10 times by volume when immersed in de-ionized water. In some forms the foam material **200** has a minimum wall thickness of about 1mm.

In use, the device is implanted within an opening to a heart. In some forms, the device is implanted prior to the implantation of a heart valve. In certain embodiments, the device is configured to create an improved implantation site for a heart valve by, for example, maintaining a substantially circular lumen **118.** In some forms, support frame **130** is configured to maintain said circular lumen for at least as long as it takes for a surgeon to deliver and implant a heart valve within the lumen **118.** In some forms, the device is attached to a heart valve prior to implantation. Such attachment may be accomplished by for example: stitching, use of an adhesive, or mechanical interlocking with the surface of the heart valve. In certain embodiments, the device is delivered and implanted concurrently with a heart valve, creating an effective seal within an opening to a heart (e.g. within the annulus). Upon implantation, the device is configured to expand upon contact with a fluid, for example blood. The device is configured to maintain a substantially circular inner lumen while the foam material **200** expands and pushes the outer surface **116** of the cuff **110** against patient tissue.

As disclosed above, certain aspects of the present invention involve foam or sponge form materials that are capable of compression to a compressed state, and resilient expansion from that compressed state. Suitable materials may include for example, expanded extracellular matrix, as described in U.S. Patent Application No. 12/488,974 to Johnson et al., which is hereby incorporated by reference. Illustratively, expansion of extracellular matrix materials can be accomplished by controlled contact with an alkaline substance. Notably, such treatment can be used to promote substantial expansion (i.e. greater than about 20% expansion) of the extracellular matrix material. In certain embodiments, it is preferred to expand the material to at least 2, at least 3, at least 4, at least about 5, or even at lest about 6 times its original bulk volume. It will be apparent to one skilled in the art that the magnitude of expansion is related to the concentration of the alkaline substance, the exposure time of the alkaline substance to the material, and temperature, among others. These factors can be varied through routine experimentation to achieve a material having the desired level of expansion, given the disclosures herein.

A collagen fibril is comprised of a quarter-staggered array of tropocollagen molecules. The tropocollagen molecules themselves are formed from three polypeptide chains linked together by covalent intramolecular bonds and hydrogen bonds to form a triple helix. Additionally, covalent intermolecular bonds are formed between different tropocollagen molecules within the collagen fibril. Frequently, multiple collagen fibrils assemble with one another to form collagen fibers. It is believed that the addition of an alkaline substance to the material as described herein will not significantly disrupt the intramolecular and intermolecular bonds, but will denature the material to an extent that provides to the material a processed thickness that is at least twice the naturally-occurring thickness. In this regard, denaturation of the collagenous material to the extent described above allows for the production of a novel collagenous matrix material. The collagenous matrix material comprises a sterile, processed collagenous matrix material derived from a collagenous animal tissue layer; the collagenous animal tissue layer has a naturally-occurring thickness and includes a network of collagen fibrils having naturally-occurring intramolecular cross links and naturally-occurring intermolecular cross links. The naturally-occurring intramolecular cross links and naturally-occurring intermolecular cross links have been retained in the sterile, processed collagenous matrix material sufficiently to maintain the sterile, collagenous matrix material as an intact collagenous sheet material, and the collagen fibrils as they occur in the intact collagenous sheet material are denatured to an extent that provides to the intact collagenous sheet material a processed thickness that is substantially greater (i.e. at least about 20% greater) than, and preferably at least twice the naturally-occurring thickness of, the collagenous animal tissue layer.

In addition to causing expansion of a remodelable collagenous material, the application of an alkaline substance can alter the collagen packing characteristics of the material. Altering such characteristics of the material can be caused, at least in part, by the disruption of the tightly bound collagenous network. A non-expanded remodelable collagenous material having a tightly bound collagenous network typically has a continuous surface that is substantially uniform even when viewed under magnification. Conversely, an expanded remodelable collagenous material typically has a surface that is quite different in that the surface is typically not continuous but rather presents collagen strands or bundles in many regions that are separated by substantial gaps in material between the strands or bundles. Consequently, an expanded remodelable collagenous material typically appears more porous than a non-expanded remodelable collagenous material. Moreover, the expanded remodelable collagenous material can be demonstrated as having increased porosity, e.g. by measuring its permeability to water or other fluid passage. The more foamy and porous structure of an expanded remodelable collagenous material can allow the material to be easily cast into a variety of foam shapes for use in the preparation of medical materials and devices. It can further allow for the compression and subsequent expansion of the material, which is useful, for example, when the material needs to be loaded into a deployment device for delivery into a patient. Once delivered, the material can expand to its original form.

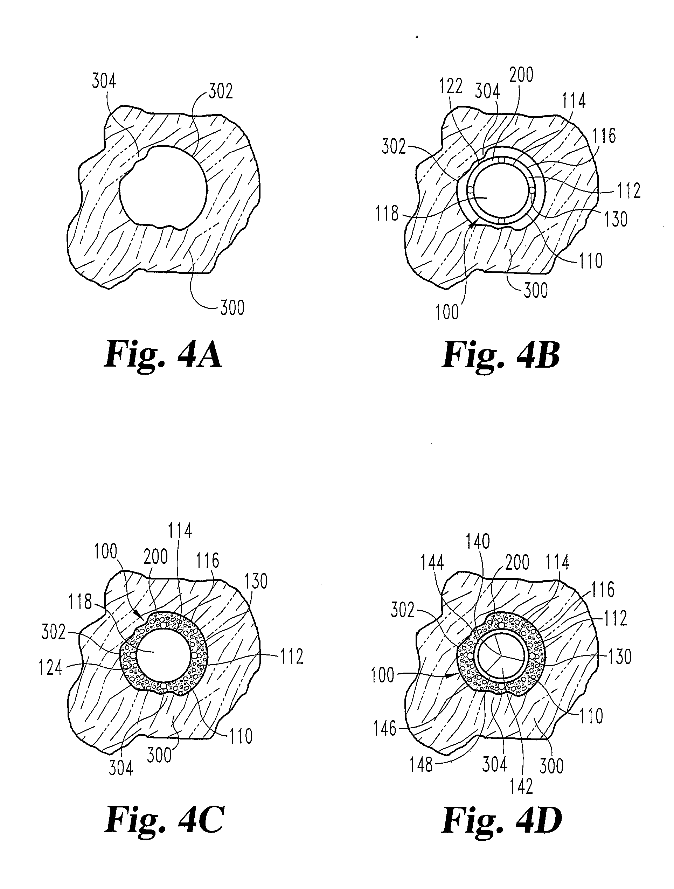

Turning now to FIGS. 4A-4D, FIG. 4A provides a top view of an expanded opening to a heart. Such expansion may occur for example by balloon dilation. As shown in FIG. 4A, opening **302** in patient tissue **300** includes one or more irregularities **304** which may represent for example, fractured calcium deposits at the implantation site and may prevent proper sealing of a percutaneously delivered heart valve resulting in perivalvular leakage. FIG. 4B illustrates device **100** within opening to a heart **302.** When delivered, cuff **110** is in a first compressed state **122** wherein tubular wall **112** has a compressed wall thickness between inner surface **114** and outer surface **116.** FIG. 4C illustrates the disclosed device after contact with a liquid, for example blood, in which foam material **200** has expanded and outer surface **116** substantially contacts patient tissue **300** sealing the periphery of opening to a heart **302.** As illustrated, lumen **118** is supported by support frame **130** in a substantially circular cross-section configured to receive a heart valve as illustrated in FIG. 4D which shows a heart valve **140** received within lumen **118.**

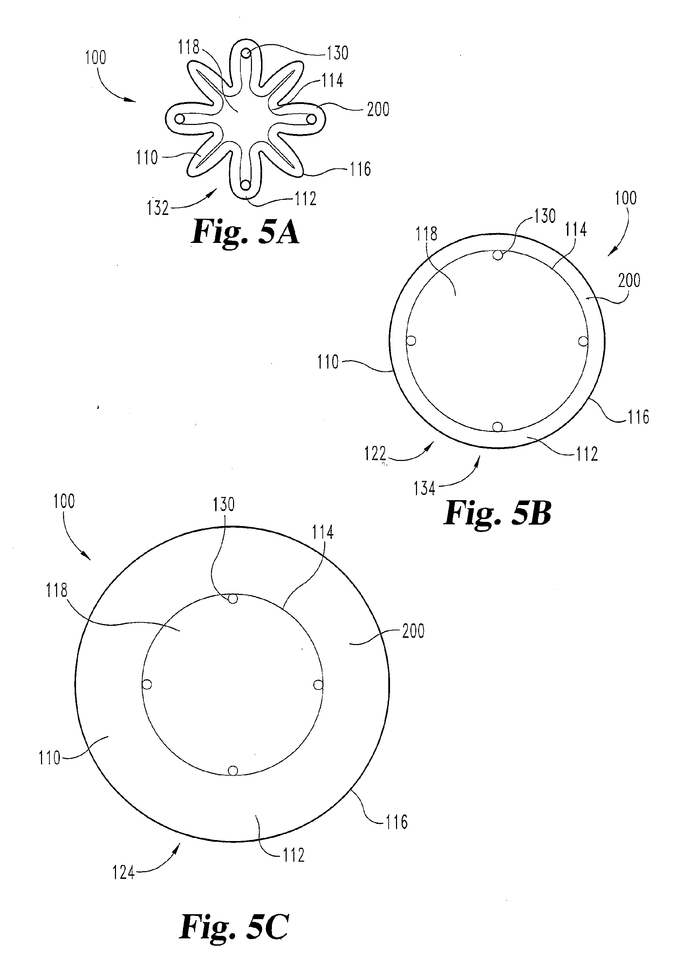

In some forms, device **100** may be collapsed to a collapsed frame position **132** as illustrated in FIG. 5A. A collapsed frame position **132** is configured to allow device **100** to pass through a cannulated device for percutaneous delivery to an opening to a heart. Support frame **130** may, for example, be resiliently deformable. In some forms device **100** may then adopt an expanded frame position **134** as shown in FIG. 5B in which support frame **130** is expanded to form an enlarged lumen **118.** As illustrated, foam material **200** is in a first compressed state **122** which upon contact with a liquid will swell to a second swelled state **124** illustrated in FIG. 5C. In some forms support frame **130** is configured to have sufficient hoop strength to resist compressive force from expansion of foam material **200** to maintain a substantially circular cross-section in lumen **118** for receiving a heart valve **140.**

In certain embodiments, device **100** includes one or more retention elements **126.** An illustrative embodiment is shown in FIG. 6. Retention elements **126** are sized and configured to retain device **100** within an opening to a heart. Retention elements **126** may, for example, comprise barbs, points, hooks, ribs, protuberances, an adhesive material, and/or other suitable surface modifications to anchor the device in an opening to a heart. In some forms retention elements **126** are supported by support member **127.** Support member **127** may, for example, be embedded within foam material **200.** In some forms retention elements **126** are supported by and/or are monolithic with support frame **130.** Retention elements **126** may be composed of, for example, poly lactic-*co*-glycolic acid or nitinol.

Expanded remodelable collagenous materials, as well as tissue extracts as described herein, are prepared, for example, from collagenous materials isolated from a suitable tissue source from a warm-blooded vertebrate, and especially a mammal. Such isolated collagenous material can be processed so as to have remodelable properties and promote cellular invasion and ingrowth. Suitable remodelable materials can be provided by collagenous extracellular matrix (ECM) materials possessing biotropic properties.

Suitable bioremodelable materials can be provided by collagenous extracellular matrix materials (ECMs) possessing biotropic properties, including in certain forms angiogenic collagenous extracellular matrix materials. For example, suitable collagenous materials include ECMs such as submucosa, renal capsule membrane, dermal collagen, dura mater, pericardium, fascia lata, serosa, peritoneum or basement membrane layers, including liver basement membrane. These and other similar animal-derived tissue layers can be expanded and processed as described herein. Suitable submucosa materials for these purposes include, for instance, intestinal submucosa, including small intestinal submucosa, stomach submucosa, urinary bladder submucosa, and uterine submucosa.

Submucosa or other ECM tissue used in the invention is preferably highly purified, for example, as described in U.S. Patent No. 6,206,931 to Cook et al. Thus, preferred ECM material will exhibit an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 5 µg/mg, more preferably less than about 2 µg/mg, and virus levels are preferably less than about 50 plaque forming units (PFU) per gram, more preferably less than about 5 PFU per gram. These and additional properties of submucosa or other ECM tissue taught in U.S. Patent No. 6,206,931 may be characteristic of the submucosa tissue used in the present invention.

In order to prepare an expanded remodelable collagenous material, the material is preferably treated with a disinfecting agent so as to produce a disinfected, expanded remodelable collagenous material. Treatment with a disinfecting agent can be done either prior to or after isolation of the remodelable collagenous material from the tissue source or can be done either prior to or after expansion. In one preferred embodiment, the tissue source material is rinsed with a solvent, such as water, and is subsequently treated with a disinfecting agent prior to delamination. It has been found that by following this post-disinfection-stripping procedure, it is easier to separate the remodelable collagenous material from the attached tissues as compared to stripping the remodelable collagenous material prior to disinfection. Additionally, it has been discovered that the resultant remodelable collagenous material in its most preferred form exhibits superior histology, in that there is less attached tissue and debris on the surface compared to a remodelable collagenous material obtained by first delaminating the submucosa layer from its source and then disinfecting the material. Moreover, a more uniform remodelable collagenous material can be obtained from this process, and a remodelable collagenous material having the same or similar physical and biochemical properties can be obtained more consistently from each separate processing run. Importantly, a highly purified, substantially disinfected remodelable collagenous material is obtained by this process. In this regard, one embodiment of the invention provides a method for preparing an expanded remodelable collagenous material. The method comprises providing a tissue source including a remodelable collagenous material, disinfecting the tissue source, isolating the remodelable collagenous material from the tissue source, and contacting the disinfected remodelable collagenous material with an alkaline substance under conditions effective to expand the remodelable collagenous material to at least about two times its original volume, thereby forming the expanded remodelable collagenous material. Upon formation of the expanded remodelable collagenous material, the material can be further processed into medical materials and/or devices, or can be stored, e.g. in high purity water at 4 °C, for later use.

Preferred disinfecting agents are desirably oxidizing agents such as peroxy compounds, preferably organic peroxy compounds, and more preferably peracids. As to peracid compounds that can be used, these include peracetic acid, perpropioic acid, or perbenzoic acid. Peracetic acid is the most preferred disinfecting agent for purposes of the present invention. Such disinfecting agents are desirably used in a liquid medium, preferably a solution, having a pH of about 1.5 to about 10, more preferably a pH of about 2 to about 6, and most preferably a pH of about 2 to about 4. In methods of the present invention, the disinfecting agent will generally be used under conditions and for a period of time which provide the recovery of characteristic, purified submucosa materials as described herein, preferably exhibiting a bioburden of essentially zero and/or essential freedom from pyrogens. In this regard, desirable processes of the invention involve immersing the tissue source or isolated remodelable collagenous material (e.g. by submersing or showering) in a liquid medium containing the disinfecting agent for a period of at least about 5 minutes, typically in the range of about 5 minutes to about 40 hours, and more typically in the range of about 0.5 hours to about 5 hours.

When used, peracetic acid is desirably diluted into about a 2% to about 50% by volume of alcohol solution, preferably ethanol. The concentration of the peracetic acid may range, for instance, from about 0.05% by volume to about 1.0% by volume. Most preferably, the concentration of the peracetic acid is from about 0.1% to about 0.3% by volume. When hydrogen peroxide is used, the concentration can range from about 0.05% to about 30% by volume. More desirably the hydrogen peroxide concentration is from about 1% to about 10% by volume, and most preferably from about 2% to about 5% by volume. The solution may or may not be buffered to a pH from about 5 to about 9, with more preferred pH's being from about 6 to about 7.5. These concentrations of hydrogen peroxide can be diluted in water or in an aqueous solution of about 2% to about 50% by volume of alcohol, most preferably ethanol.

With respect to the alkaline substance used to prepare an expanded remodelable collagenous material, any suitable alkaline substance generally known in the art can be used. Suitable alkaline substances can include, for example, salts or other compounds that that provide hydroxide ions in an aqueous medium. Preferably, the alkaline substance comprises sodium hydroxide (NaOH). The concentration of the alkaline substance that is added to the material can be in the range of about 0.5 to about 4 M. Preferably, the concentration of the alkaline substance is in the range of about 1 to about 3 M. Additionally, the pH of the alkaline substance will typically range from about 8 to about 14. In preferred embodiments, the alkaline substance will have a pH of from about 10 to about 14, and most preferably of from about 12 to about 14.

In addition to concentration and pH, other factors such as temperature and exposure time will contribute to the extent of expansion. In this respect, it is preferred that the exposure of the remodelable collagenous material to the alkaline substance is performed at a temperature of about 4 to about 45 °C. In preferred embodiments, the exposure is performed at a temperature of about 25 to about 37 °C, with 37 °C being most preferred. Moreover, the exposure time can range from about several minutes to about 5 hours or more. In preferred embodiments, the exposure time is about 1 to about 2 hours. In a particularly preferred embodiment, the remodelable collagenous material is exposed to a 3 M solution of NaOH having a pH of 14 at a temperature of about 37 °C for about 1.5 to 2 hours. Such treatment results in the expansion of a remodelable collagenous material to at least about twice its original volume. As indicated above, these processing steps can be modified to achieve the desired level of expansion.

In addition to an alkaline substance, a lipid removal agent can also be added to a remodelable collagenous material either prior to, in conjunction with, or after the addition of the alkaline substance. Suitable lipid removal agents include, for example, solvents such as ether and chloroform, or surfactants. Other suitable lipid removal agents will be apparent to those of ordinary skill in the art. Accordingly, the lipid removal agents listed herein serve only as examples, and are therefore in no way limiting.

In preferred embodiments, the expanded remodelable collagenous materials, as well as tissue extracts containing bioactive components that can optionally be added to an expanded remodelable collagenous material, are sterilized using conventional sterilization techniques including tanning with glutaraldehyde, formaldehyde tanning at acidic pH, ethylene oxide treatment, propylene oxide treatment, gas plasma sterilization, gamma radiation, and peracetic acid sterilization. A sterilization technique which does not significantly alter the remodelable properties of the expanded remodelable collagenous material is preferably used. Moreover, in embodiments where the expanded remodelable collagenous material includes a native or non-native bioactive component, the sterilization technique preferably does not significantly alter the bioactivity of the expanded remodelable collagenous material. Preferred sterilization techniques include exposing the extract to peracetic acid, low dose gamma irradiation (2.5 mRad) and gas plasma sterilization.

All publications and patent applications cited in this specification are herein incorporated by reference as if each individual publication or patent application were specifically and individually indicated to be incorporated by reference. Further, any theory, mechanism of operation, proof, or finding stated herein is meant to further enhance understanding of the present invention, and is not intended to limit the present invention in any way to such theory, mechanism of operation, proof, or finding. While the invention has been illustrated and described in detail in the drawings and foregoing description, the same is to be considered as illustrative and not restrictive in character, it being understood that only selected embodiments have been shown and described and that all equivalents, changes, and modifications that come within the spirit of the inventions as defined herein or by the following claims are desired to be protected.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 61/924,870, from which this application claims priority, and in the abstract accompanying this application are incorporated herein by reference.

## Claims

1. A device for reducing perivalvular flow associated with percutaneously delivered heart valves, the device including:
a support frame;
a cuff including a foam material, said cuff comprising a tubular wall with a minimum thickness of about 1mm, said tubular wall defined by an inner surface and an outer surface, wherein said tubular wall is deformable to a first compressed state and is swellable upon contact with a liquid to a second swelled state; and
a lumen defined by said inner surface, said lumen sized and configured to receive an implantable heart valve, wherein said support frame has a hoop strength sufficient to maintain said lumen with a substantially circular cross section.

2. A device according to claim 1, wherein:
said foam material expands at least 3 times by volume when saturated with distilled water.

3. A device according to claim 1 or 2, wherein:
said foam material is a synthetic polymer, an extracellular matrix foam material or a cross-linked extracellular matrix foam material.

4. A device according to any preceding claim, wherein:
said tubular wall is sized and configured such that in said swelled state said cuff pushes against patient tissue.

5. A device according to any preceding claim, wherein:
said device is expandable from a collapsed position configured for passage through a cannulated device, to an expanded position configured for placement within an opening to a heart.

6. A device according to any preceding claim, wherein:
said support frame is configured to push against said inner surface.

7. A device according to any preceding claim, wherein:
said support from is embedded within said foam material.

8. A device according to any preceding claim, wherein:
said support frame is composed of Nitinol and/or is bioresorbable.

9. A device according to any preceding claim, including:
one or more retention elements configured to secure said device in an opening to a heart.

10. A device according to claim 10, wherein:
said retention elements include barbs on said outer surface.

11. A device for reducing perivalvular flow associated with percutaneously delivered heart valves, the device including:
a cuff having an inner surface and an outer surface, said inner surface defining a lumen, said cuff including a foam material, wherein said foam material is deformable to a first compressed state and is swellable upon contact with a liquid to a second swelled state, said cuff sized and configured such that in said swelled state said outer surface pushes against an opening to a heart; and
a heart valve receivable within said lumen.

12. A device according to claim 11, wherein:
said foam material expands at least 3 times when saturated in distilled water.

13. A device according to claim 11 or 12, wherein:
said foam material is an extracellular matrix foam material.

14. A device according to claim 11, 12 or 13, including:
a support frame configured to support said lumen prior to insertion of said heart valve.

15. A device according to claim 14, wherein:
said support frame is bioresorbable and/or comprises nitinol.

16. A device according to any one of claims 11 to 15, including:
one or more retention elements configured to secure said device within the opening to a heart.

17. A device according to claim 16, wherein:
said retention elements include barbs attached to said cuff.
